# EUROPEAN PATENT SPECIFICATION

(11) **EP 1 750 686 B1**
(45) Date of publication and mention of the grant of the patent: **05.04.2017**
(21) Application number: 05742563.9
(22) Date of filing: 04.05.2005
(51) Int. Cl.: A61K 31/19, A61P 25/08

(54) **Treatment of acute intractable seizures with (R)-3-hydroxybutyrate**
Behandlung von schwerbehandelbaren epileptischen Anfällen mit (R)-3-Hydroxybutyrat
Traitement de crises épileptiques réfractaires avec (R)-3-hydroxybutyrate

(30) Priority: 07.05.2004 GB 0410266
(43) Date of publication of application: 14.02.2007
(73) Proprietor: BTG International Limited, London EC4M 7RD (GB)
(72) Inventor: MARTIN, Keith Frank BTG International Limited, London EC4M 7SB (GB); HEAL, David John RenaSci Consultancy Limited, Nottingham NG1 1GF (GB); JAGGER, Elizabeth RenaSci Consultancy Limited, Nottingham NG1 1GF (GB)
(74) Representative: BTG plc Intellectual Property Group
(86) International application number: PCT/GB2005/001699
(87) International publication number: WO 2005/107724

(56) References cited:
- EP-A- 1 188 437
- WO-A-00/28985
- WO-A-98/41200
- WO-A-02/062327
- NAKAMURA SHIGERU ET AL: "Protective effect of D-beta-hydroxybutyrate on corneal epithelia in dry eye conditions through suppression of apoptosis." IOVS, vol. 44, no. 11, November 2003 (2003-11), pages 4682-4688, XP009051914
- KOUSTOVA ELENA ET AL: "Ketone and pyruvate Ringer's solutions decrease pulmonary apoptosis in a rat model of severe hemorrhagic shock and resuscitation." SURGERY (ST LOUIS), vol. 134, no. 2, August 2003 (2003-08), pages 267-274, XP002339609 ISSN: 0039-6060
- ZOU ZHITIAN ET AL: "dl-3-Hydroxybutyrate administration prevents myocardial damage after coronary occlusion in rat hearts." AMERICAN JOURNAL OF PHYSIOLOGY, vol. 283, no. 5 Part 2, November 2002 (2002-11), pages H1968-H1974, XP009051908 ISSN: 0002-9513

## Description

The present invention relates to a material for use in treating patients with acute intractable seizures, wherein the treatment is initiated after onset of seizures to arrest apotosis, a condition that is resistant to treatment with conventional anti-epileptic drugs, such that they do not suffer long term cerebral impairment.

The ketogenic diet is an established non-pharmacologic treatment for controlling, eg. reducing or eliminating, seizures in patients with pharmaco-resistant (or intractable) epilepsy (Vining EPG (1999)). The ketogenic diet is a high fat, low protein diet which is believed to alleviate seizures by forcing the body to use ketone bodies as the predominant fuel source. However, a major disadvantage of the diet is that seizure control is lost upon ingestion of carbohydrates, which can make compliance with the diet difficult, particularly for children. In addition, there are problems associated with a high calorific intake. Seizure control appears to correlate with blood levels of (R)-3-hydroxybutyrate (β-OHB or BHB herein) (Freeman et al (1998), Bough and Bough et al (1999)) suggesting a direct role for this ketone body in limiting seizure activity.

It is known to use (R)-3-hydroxybutyrate, its salts and esters to reduce cerebral edema and reduce volume of cerebral infarct on challenge such as stroke or head trauma or metabolic poison or abnormality (see eg. Veech US 6316038 and Hiraide et al EP 01780123 B: such use may be acute in effect and the active agent may be adminsitered immediately after stroke or trauma.

It is known that the ketone bodies, acetoacetate and D-β-hydroxybutyrate ((R)-3-hydroxybutyrate), can be used to treat seizures. Whereas early experiments show that acetoacetate and its sodium salt reduced seizures induced with thujone, they could not replicate this with (R)-3-hydroxybutyrate (see H Keith et al Arch Neurol Psych 29. 148-154 (1933) and H Keith et al J Pharm Exp Ther 44 449, (1931)). However, Veech (WO 98/41201) and Niesen (WO 00/28985) have since taught how (R)-3-hydroxybutyrate and its salts and esters can be used to treat chronic epilepsy. Niesen evidences antiepileptic properties of 3-hydroxybutyrate using pentylenetetrazol (PTZ) as convulsion inducing agent whereby tonic-clonic seizures were induced in rats and in treatment groups seizure duration and mortality rate were both reduced. These results were mirrored in rats put on a ketogenic diet. All treatments were initiated three weeks before seizures were induced.

Most patients with epilepsy achieve complete seizure control by therapeutic intervention with antiepileptic drugs. However, approx 25 - 30 % of epilepsy patients remain intractable to current anticonvulsant treatment. Intractable epilepsy is thought to develop following an initial seizure event early in life, such as a febrile seizure or status epilepticus (Sutula T et al (1989) and Babb TL et al (1991)). This seizure activity leads to neuronal cell death and subsequent epileptogenesis that eventually develops into pharmaco-resistant epilepsy.

Status epilepticus is accompanied by abnormal electrical activity involving all areas of the cortex, sometimes simultaneously and sometimes spreading from a focal area of electrical abnormality. Traditionally, status epilepticus was defined as 30 minutes of continuous seizure activity or a series of seizures without return to full consciousness. Pathology suggest that a shorter period of seizure activity causes neuronal injury and that seizure self-termination becomes unlikely after 5 minutes.

Although cerebral metabolic demand increases, cerebral blood flow and oxygenation are thought to be preserved or even elevated. Significantly, neuronal loss after focal or generalized status epilepticus is linked to the abnormal neuronal discharges and not simply to the systemic effects, with the hippocampus being especially vulnerable to damage. Whilst most seizures terminate spontaneously, current treatments include drugs with significant side effects such as benzodiazepines, barbituates and anaesthetics. Status remains a serious inadequately treated condition, with approximately 50,000-200,000 cases of status epilepticus occuring per year in the US alone with an overall mortality rate of about 20%.

It is further known that cells may die *inter alia* directly due to the effects of mechanical, chemical or biological injury, or may 'self desctruct' by so called programmed cell death or process of apoptosis. This is a complex process that has been noted to involve one or more of shrinking, development of bubble-like surface bodies, degredation of nuclear chromatin, destruction of mitochondria with release of cytochrome c, cellular fragmentation and exposure of immuno-reactive phosphatidylserine with resultant attack by macrophages and dendricytes. Thus, whereas acute mechanical, chemical or biological injury results in acute cell death, apoptosis can take a number of days. If apoptosis could be treated by safe and reliable means, many disease states could be managed more successfully.

Apoptotic death can be triggered by a wide variety of stimuli, and not all cells necessarily will die in response to the same stimulus. Human diseases, including cancer, viral infections, autoimmune diseases, neurodegenerative disorders, and AIDS (acquired immunodeficiency syndrome) all have apoptotic pathogenesis to some extent. Among the more studied death stimuli is DNA damage (by irradiation or drugs used for cancer chemotherapy), which in many cells leads to apoptotic death via a pathway dependent on p53. Some hormones such as corticosteroids lead to death in particular cells (e.g., thymocytes), although other cell types may be stimulated. Some cells types express Fas, a surface protein which initiates an intracellular death signal in response to crosslinking. In other cases cells appear to have a default death pathway which must be actively blocked by a survival factor in order to allow cell survival.

The present inventors have now determined that ketosis, particularly produced by a ketogenic drugs substance after onset of status epilepticus, is capable of protecting the cerebral function of a patient by reducing, and even preventing, apoptosis induced by damage from the abnormal neuronal discharge. Although some damage will be expected due to necrosis induced in the very early stages of the seizure, they have unexpectedly found that significant apoptosis, which ketosis is capable of eliminating, occurs in the days following the seizure.

Thus in a first aspect of the present invention there is provided a ketogenic material selected from (R)-3-hydroxybutyrate and its salts, esters and oligomers for use in the treatment of acute intractible seizures wherein the treatment is intiated after onset of the seizures to arrest apoptosis.

Preferably the treatment is for status epilepticus.

The treatment produces a ketosis in which levels of one or both of acetoacetate and (R)-3-hydroxybutyrate concentrations in the blood of the subject are raised. Preferably the total concentration of these 'ketone bodies' in the blood is elevated above the normal fed levels to between 0.1 and 30mM, more preferably to between 0.3 and 15mM, still more preferably to between 0.5 and 10mM and most preferably to between 3 and 8mM. For the purpose of maximising levels of such compounds in the CNS it is desirable to saturate the transporter through which (R)-3-hydroxybutyrate crosses the blood brain barrier: this occurring at between 3 and 5mM.

Particular materials are known from the following references as set out in Table 1 below. Doses and formats are as described in the documents identified in the table. Typically the amount of ketogenic material required can be determined by measuring blood levels directly using a meter such as the Medisense Precision Extra (MedisenseInc, 4A Crosby Drive Bedford, MA 01730); BioScanner 2000 (formerly called the MTM BioScanner 1000) from Polymer Technology Systems Inc. Indianapolis, Indiana. In this manner the amount of ketosis derived from a set dose may be ascertained, and that dose iterated to suit the individual.

Typical dose ranges for example might be in the range 5 to 5000mg/kg body weight, particularly for an (R)-3-hydroxybuytrate containing material such as oligomeric (R)-3-hydroxybuytrate or its esters with, eg, glycerol or (R)-butan-1,3-diol, more preferably 30 to 2000mg/kg body weight, most preferably 50 to 1000mg/kg body weight per day.

The condition of acute intractible seizures, necessitates that the compound be given, sometimes at least, when the patient is unable to volutarily admit materials by mouth. Thus where the route of adminstration is oral, it must be via a gastric feeding tube. More conveniently the compound or compounds will be administered as a parenteral solution, intravenously, interperitoneally or by some other systemic route.

Suitable ketogenic materials are described in the prior art in Table 1.

Where these are not soluble in aqueous solutions, they may be provided as emulsions. Dosage forms and dosages may be as above or as described in any of the references.

The present invention will now be described by way of the following non-limiting Experimental, Examples and Figures. Further embodiments falling into the scope of the claims herein will occur to those skilled in the light of these.

### FIGURES

Figure 1: shows the effect of administration of sodium (R)-3-hydroxybutyrate (KTX0101) on kainic acid induced neuronal cell death at 24 hours post challenge
Figure 2: shows propidium iodide uptake in hippocampal organotypic cultures 24 hours post challenge.
Figure 3: shows the effect of administration of sodium (R)-3-hydroxybutyrate (KTX0101) on kainic acid induced neuronal cell death at 7 days.
Figure 4: shows propidium iodide uptake in hippocampal organotypic cultures at 7 days following treatment.

### EXPERIMENTAL

KTX0101 (as used herein below) is the sodium salt of (R)-3-hydroxybutyrate (akaBHB or D-β-hydroxybutyrate).

A seizure-induced neuronal cell death *in vitro* experimental model of status epilepticus was provided as follows. The neuroprotective potential of (R)-3-hydroxybutyrate sodium salt (BHB) (0.3 mM, 1 mM and 3 mM) was assessed by propidium iodide uptake into hippocampal organotypic cultures administered 1 µM KA for 24 h to induce experimental status epilepticus. Neuronal cell death is already observed at 24 h subsequent to KA-application therefore, it was of no value to administer to the cultures at this time-point. Test compound was, therefore, added to the cultures before significant neuronal cell death occurred. Thirty minutes of status epilepticus is believed to demarcate the transition point at which seizure-induced neuronal injury takes place in patients (Lowenstein DH (1999); hence BHB, as KTX0101, was administered 30 mins after addition of KA with neuronal cell death assessed 24 h later.

Since there is substantial evidence to indicate that BHB plays a key role in modulating seizure activity, this intervention may be therapeutically valuable in preventing further epileptogenesis and the associated structural damage that leads to the subsequent development of pharmaco-resistant epilepsy. Therefore, the neuroprotective potential of BHB was also assessed following incubation over a 7 day period following KA-induced status epilepticus.

### Methodology Animals and environment

Two time-mated female Wistar rats were obtained from Charles River, UK and housed on a 12 h/12 h light/dark cycle (lights on at 07.30 h), at an ambient temperature of 21°C and 55 % humidity. Food and water were available *ad libitum.*

All procedures were performed in strict accordance with Home Office Guidelines, Home Office Schedule 1 Procedure.

### Hippocampal organotypic cultures

All culture procedures were carried out in a laminar flow hood using aseptic techniques and sterilised equipment and solutions. Throughout the experimental procedure no antibiotics were utilised.

Hippocampal organotypic slice cultures were prepared according to the Gähwiler roller-tube method (Gähwiler BH (1981) and Gähwiler et al (1997)) from nine rat pups aged between 5 - 7 post-partem.

Rat pups were killed by cervical dislocation, their brains rapidly removed and placed in a Petri dish. The meninges were removed from the caudal section of the cortex and then the brain was located with the ventral surface uppermost. Two transverse cuts were made, the first, at the level of the cerebellum to remove the brainstem, and the second, at the level of the optic chiasm to remove the forebrain. The cerebellum, midbrain and hindbrain were lifted upwards from the surrounding cortical tissue leaving one hippocampus embedded within each cortical hemisphere. An incision was made down the midline to separate the two cortical hemispheres. To separate the hippocampus from the surrounding cortical tissue three incisions were made around the hippocampus and then it was rolled out from the underlying tissue. The isolated hippocampus was then placed in a drop of ice-cold Gey's balanced salt solution containing 0.5 % D-glucose (GBSS+G). The procedure was then repeated to isolate the remaining hippocampus. The whole procedure was performed using a Swift dissecting microscope and took between 10 -15 mins thereby minimizing tissue deterioration.

The hippocampi were transferred to a McIlwain tissue chopper and sectioned into a series of 400 µm thick slices. After sectioning, the hippocampi were placed in ice-cold GBSS+G and the slices gently teased apart. The slices were washed once in GBSS+G and the integrity of the slices checked under a higher magnification. If the slices had any tears, an opaque appearance or the cell body layers were not easily visible, they were discarded. A healthy slice has a yellow-brown colouration without any blackening in the cell body regions.

To adhere the hippocampal slice to a coverslip (Agar Scientific, UK), 20 µl of chicken plasma was placed in the middle of the coverslip followed by a hippocampal slice. The plasma was spread thinly over the surface of the coverslip, to ensure the slice was well coated. Then, 30 µl of thrombin (75 units/ml activity) was placed over the slice and mixed thoroughly with the chicken plasma. The plasma-thrombin mixture was left for approximately 60 s until a clot of jelly-like consistency had formed. The coverslip was then placed in a test-tube (Nunclon, UK) which contained 750 µl of pre-warmed culture media (37°C). Culture media consisted of 50 % basal media Earles, 25 % Hank's balanced salt solution, 25 % heat inactivated horse serum, 100 mM L-glutamine and 0.5 % D-glucose. The test tubes were sealed with a screw top lid and placed in a custom made roller-drum (Medical School Workshop, University of Nottingham) at an angle of 5°, rotating at 10 revolutions per hour in a dry incubator at 37°C.

After 48 h in vitro, 500 µl of media was removed and the cultures were incubated in culture media containing a combination of anti-mitotics, which consisted of 5-fluorodeoxyuridine, cytosine-β-D-arabino-furanoside (ARA-C) and uridine each at a concentration of 1 µM. After a further 24 h, 500 µl of media was removed and replaced with 500 µl of new culture media containing no anti-mitotics.

### Administration of drugs

Cultures used to evaluate the neuroprotective potential of BHB were between the ages of 8 - 20 days in vitro. Sterile conditions were maintained throughout the experimental period, which enabled the determination of neuronal cell death in the same cultures over time which is not possible *in vivo.* The pattern of neuronal cell death was observed in the CA1 region of hippocampal organotypic cultures using a confocal microscope at the University of Nottingham.

Five different experimental groups were employed (Table 2) and the pattern of neuronal cell death was evaluated at 24 h and 7 d following treatment. The cultures in all groups were incubated with propidium iodide at a concentration of 2 µM. The cultures in the control group were administered fresh medium containing propidium iodide only, 24 h prior to assessment with the confocal microscope. KA-treated cultures were incubated with fresh media containing 1 µM KA plus propidium iodide for 24 h. The cultures in the remaining 3 groups were incubated for 30 mins with fresh media containing KA (1 µM) and propidium idodide, prior to the addition of KTX0101 at concentrations of 0.3, 1, or 3 mM. Cultures were examined 24 h hours later using a confocal microscope. The control and KA-treated cultures were then incubated with fresh media to ensure washout of residual propidium iodide and KA. The KTX0101-treated cultures were incubated with fresh media containing appropriate concentrations of KTX0101. After 6 d, propidium iodide was added to all cultures in the five experimental groups before examination under the confocal microscope at 7 d post-treatment.

### Assessment of neuronal cell death

Detection of seizure-induced neuronal cell death was assessed by visualization of propidium iodide uptake into dead cells. Propidium iodide is a polar compound that is excluded from intact cell membranes and is non-toxic to neurones. It rapidly enters cells following damage to the membrane and binds to the nucleic acids in the nuclei and cytoplasm, rendering a bright red fluorescence in them when viewed with a confocal microscope.

Propidium iodide uptake was visualised using a multi-tracking confocal laser-scanning microscope (Zeiss, UK) with an Argon 488 and HeMe 543 laser line on an inverted fluorescent microscope (Axiovert 100M, Zeiss, UK). Any dead cells in the CA1 region of the hippocampus were visualised at a magnification of x20 and a wavelength of 543nm. The magnification used was sufficient to capture the whole CA1 region in one view. Optical slices were collected sequentially at 2µM intervals in the z-direction. A total of 14 sequential sections were scanned and the images were displayed as maximum intensity projections of the 14 sections combined using the associated image capture software (LSM browser 510 version 3.2; Zeiss, UK).

Densitometric measurements of the cellular uptake of propidium iodide (indicated by red fluorescence) to quantify neuronal cell death were undertaken on the scanned images using Qwin Pro software (version 3.1; Leica, UK) and the percentage area of red fluorescence was calculated relative to the total area of the scanned image.

### Reagents

Gey's balanced salt solutions, basal media Earles, Hank's balanced salt solution, heat inactivated horse serum and 100 mM L-glutamine were purchased from Gibco Life Technologies (UK). Chicken plasma was obtained from Cocalico Biologicals (USA). Thrombin, 5-fluorodeoxyuridine, cytosine-β-D-arabino-furanoside, uridine, propidium iodide and KA were purchased from Sigma Chemical Company (UK). BHB, which is the sodium salt of D-β-hydroxybutyrate was synthesised by Lonza Ltd, Basel (Switzerland; Batch No.4). Stock solutions of propidium iodide, kainic acid and KTX0101 were prepared in Hank's balanced salt solution.

### Statistical analysis

The percentage area of red fluorescence was averaged for each experimental group and the values expressed as the mean ± S.E.M for *n* = 12-13 hippocampal organotypic cultures.

A one-way analysis of variance (ANOVA) followed by Dunnett's multiple comparisons test (two-tailed) was used to compare control cultures versus the KA-treated cultures and the cultures treated with a combination of KA and BHB. A P value of <0.05 was considered statistically significant.

To test for a neuroprotective effect of BHB at each time-point (24 h and 7 d) following KA-induced status epilepticus, a one-way analysis of variance (ANOVA) followed by Dunnett's multiple comparisons test (two-tailed) was used to compare the KA-treated cultures versus BHB-treated cultures. A P value of <0.05 was considered statistically significant.

### RESULTS

### The effect of BHB administration on kainic acid-induced status epilepticus at 24 h

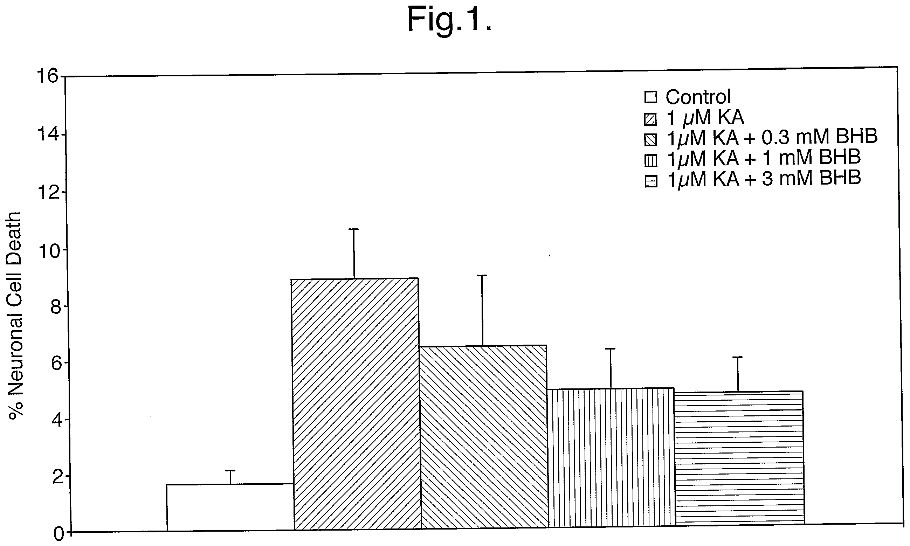

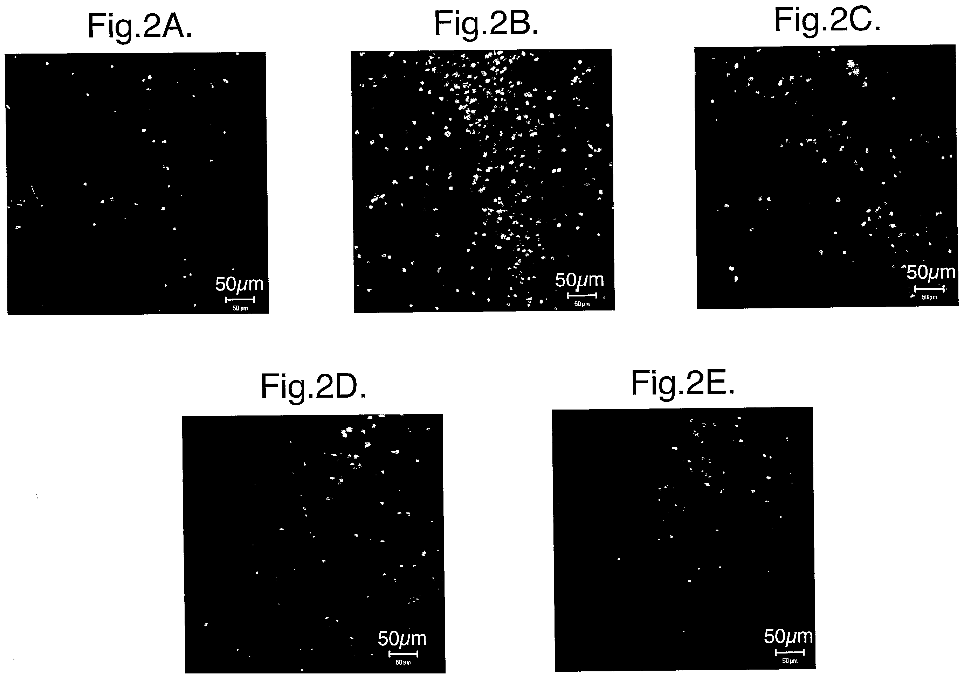

The effects of BHB addition to protect against KA-induced neuronal cell death in the CA1 region of hippocampal organotypic cultures at 24 h are shown in Table 2 and Figure 1 (*n* = 12-13). Representative digital images taken from the confocal microscope showing the profile of propidium iodide uptake in the CA1 region from each treatment group are shown in Figure 2.

KA (1 µM for 24 h) caused a significant increase in propidium iodide uptake in KA-treated cultures (8.89 ± 1.78 %, P<0.05) compared with control cultures (1.68 ± 0.49 %) (Table 2A, Figure 1 and Figures 2A and 2B).

Propidium iodide uptake did not significantly differ between control cultures and cultures incubated with a combination of KA (1 µM for 24 h) plus BHB (0.3, 1.0 or 3.0 mM for 24 h) (Table 2A, Figure 1 and Figures 2A, 2C, 2D and 2E).

No significant alteration in propidium iodide uptake was observed between KA-treated cultures and cultures co-incubated with KA (1 µM for 24 h) plus BHB (0.3, 1.0 or 3.0 mM for 24 h) (Table 2B, Figure 1 and Figures 2B, 2C, 2D and 2E).

### The effect of BHB administration on kainic acid-induced status epilepticus at 7 days

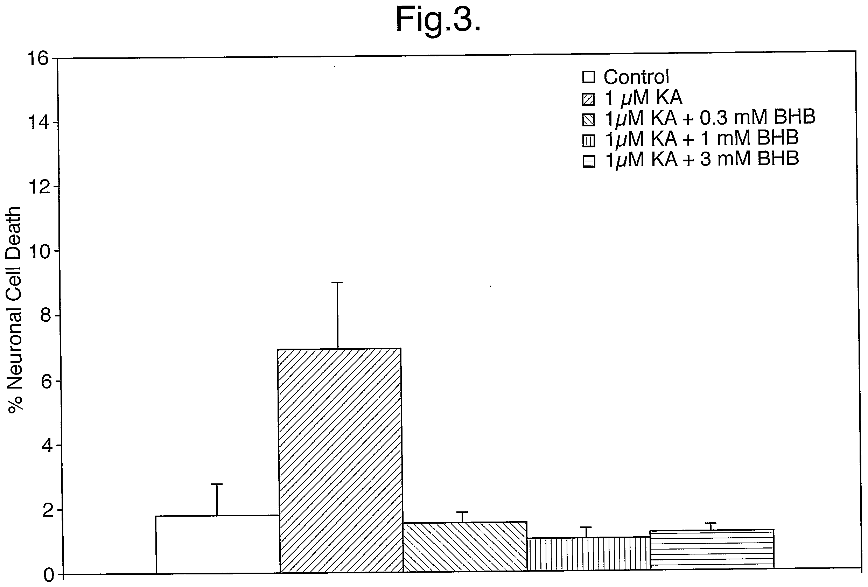

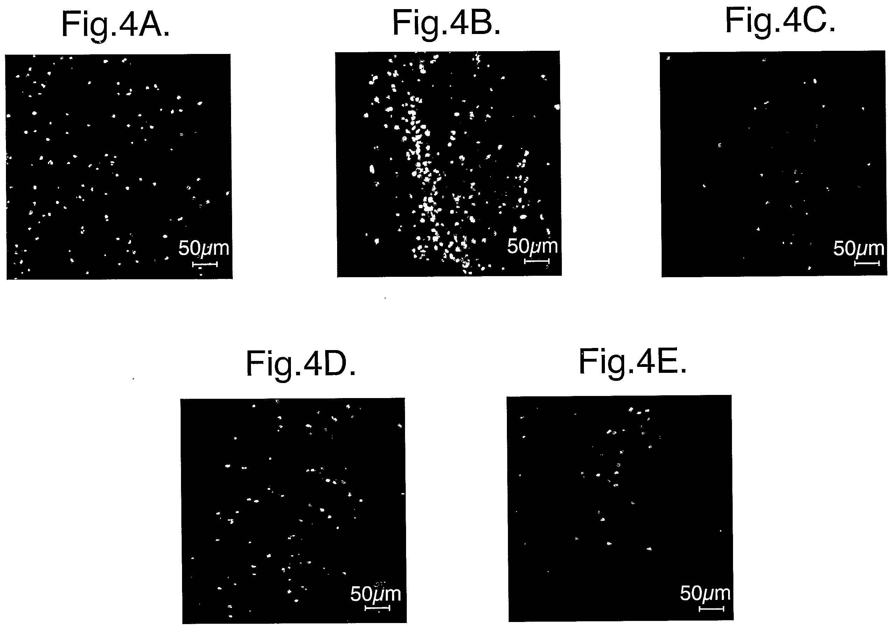

The effects of BHB addition for 7 days to prevent KA-induced neuronal cell death are shown in Table 3 and Figure 3 (*n* = 12-13). Representative digital images, taken on the confocal microscope showing the profile of propidium iodide uptake in the CA1 region of hippocampal organotypic cultures from each treatment group are shown in Figure 4.

Compared to control cultures (1.81 ± 0.99%), a significant increase in propidium iodide uptake was observed in cultures treated with KA alone (1 µM for 24 h), 7 d previously (6.97 ± 2.03 %, *P*<0.001) (Table 3A, Figure 3 and Figures 4A and 4B).

Propidium iodide uptake did not significantly differ between control cultures and cultures incubated with a combination of KA (1 µM for 24 h, 7 d previously) plus KTX0101 (0.3, 1.0 or 3.0 mM for 7 d) (Table 3A, Figure 3 and Figures 4A, 4C, 4D and 4E).

Incubation with BHB (0.3, 1.0 and 3.0 mM) for 7 d following KA-induced status epilepticus (1 µM KA for 24 h) caused a significant decrease in propidium iodide uptake compared to KA-treated cultures alone (6.97 ± 2.03 %, *P*<0.001) (Table 3B, Figure 3 and Figures 4B, 4C, 4D and 4E). Propidium iodide uptake in the BHB-treated cultures decreased to levels not significantly different from those observed in control cultures (1.81 ± 0.99 %). BHB (0.3, 1 and 3 mM) reduced propidium iodide uptake to 1.54 ± 0.33% (*P*<0.001), 1.05 ± 0.29 % (*P*<0.001) and 1.25 ± 0.21 % (*P*<0.001) respectively.

### Discussion

The experimental example above illustrates how administration of sodium salt of (R)-3-hydroxybutyrate during an episode of status epilepticus to inhibit neuronal cell death could be of therapeutic benefit to prevent the development of intractable epilepsy.

In an attempt to elucidate the neuroprotective potential of BHB, the inventors have examined the effects of 24 h and 7 day administration to prevent KA-induced neuronal cell death by measuring the cellular uptake of propidium iodide in the CA1 region of hippocampal organotypic cultures. Three concentrations of BHB (0.3, 1, and 3 mM) were investigated to elucidate the optimum therapeutic concentration for controlling status epilepticus. This range of concentrations was chosen based on reported BHB levels in patients, where seizure control was observed when blood BHB levels were less than 6 mM¹.

The rate of neuronal cell death in control cultures was similar over the 24 h and 7 day experimental period (1.68 and 1.81 % after 24 h and 7 d, respectively) illustrating that the cultures remained viable throughout the study period and that any changes observed were attributed either to a neuroprotective or neurotoxic effect of the administered drugs.

Under the present experimental conditions, KA-induced status epilepticus (1 µM for 24 h) caused neuronal cell death in the CA1 region of hippocampal organotypic cultures. A significant elevation in the percentage of dead neuronal cells (8.89 %) was observed 24 h following application of KA, which decreased by only 1.92 %, 7 d later, when KA was no longer present in the medium. These data are in accordance with in vivo studies, where a relatively small percentage of cell loss was observed in the CA1 pyramidal cell layer following KA-induced status epilepticus (Esclapez M et al (1999) and Fujikawa DG et al (2000)).

There were concentration-dependent decreases in KA-induced neuronal cell death (8.89 %) following 24 hours incubation of BHB at concentrations of 0.3 (6.48 %), 1 (4.89 %) and 3 mM (4.72 %); however, none of these effects were statistically significantly different from the KA-treated cultures.

An anti-apoptotic effect of BHB was apparent in cultures incubated with BHB for 7 days following KA-induced status epilepticus. The percentage of dead cells observed was similar to control values (1.81 %) and independent of the concentration evaluated (1.54, 1.05 and 1.25 % at 0.3, 1 and 3 mM, respectively). In KA-treated cultures after 7 days, the percentage of cell death was 6.97 %.

A difference in the effect of BHB at 24 hours and 7 days following experimental status epilepticus could be attributed to the prevention of neuronal cell death by two distinct cellular mechanisms, necrosis and apoptosis. Both apoptotic- and necrotic-mediated cell death have been extensively reported following KA application *in vitro* and *in vivo* (see references 13-21) and are a rapidly occurring form of cell death, that can be detected over the first few days following KA exposure. Injection of KA (i.p.) to rats, which induced status epilepticus for a minimum of 60 mins, caused DNA fragmentation in the hippocampus. This hallmark of apoptosis was detectable as early as 8 hours following KA administration and reached a maximum expression 48 h later (Cheung N et al (1998)),. Necrosis is detected earlier than apoptosis, acidophilic neurones, which are a characteristic feature of necrosis have been detected 40 mins following status epilepticus (Fujikawa DG (2000).

Necrosis is characterised by a concomitant disruption of ionic homeostatic processes, rapid energy depletion and a loss of membrane integrity. Due to the ultimate breakdown of the plasma membrane, the cellular contents are released into the extracellular fluid resulting in an intense inflammatory response from which cell debris can persist for months (Arends MJ and Wyllie AH (1991)). In contrast to necrosis, membrane integrity is not lost in apoptosis, and thus, no inflammatory response is elicited. Apoptotic cells are rapidly removed *in vivo* over the subsequent few days through phagocytosis. However, *in vitro* phagocytosis appears to be less efficient at removing apoptotic bodies (Leist et al (1995)), and so a loss of membrane integrity is observed in the latter stages of apoptosis, enabling propidium iodide, which is a membrane impermeable nuclear dye to label apoptotic cells before the cells are removed by phagocytosis. In this study, the cell death observed at 24 hours and 7 days can be attributed to a combination of apoptosis and necrosis. Thus, the neuroprotective action of BHB is likely to result from a reduction of apoptosis and necrosis following KA-induced status epilepticus.

In the present study, the effects of BHB to prevent seizure-induced neuronal cell death following status epilepticus was examined only over a relatively short period of time. However, there are more, far-reaching consequences with respect to the longer-term neuroprotective effect of BHB to implicate this compound in the prevention of epileptogenesis. In a study undertaken by Muller-Schwartz and co-workers (1999), adult rats were either fed the ketogenic diet or standard rat chow two days following KA-induced status epilepticus. All rats fed the ketogenic diet were ketotic throughout the duration of dietary treatment as elucidated by BHB levels. After 8 wks of treatment, a reduction in spontaneous seizure activity and mossy fibre sprouting in the dentate gyrus was observed in the ketogenic diet fed rats. In addition, significantly fewer CA1 population spikes were evoked by Schaffer collateral stimulation in the slices from the rats on the ketogenic diet, suggesting that the KA-induced hyperexcitability is ameliorated by the ketogenic diet. It is well documented that abberant mossy fibre sprouting occurs as a consequence of neuronal cell death, to reinnervate the deafferented dentate gyrus (references 10, 11, 25-27). The functional significance of the lesion-induced sprouting is that it leads to the formation of recurrent excitatory networks which contribute to the hyperexcitability and the eventual development of chronic recurrent seizures which become medically intractable to anticonvulsant treatment.

In hippocampal organotypic cultures, a similar situation was also observed, but in the CA1 region. KA-induced status epilepticus caused neuronal cell death that led to lesion-induced neuronal sprouting, which was accompanied by the development of epileptiform activity²⁸. By preventing neuronal cell death following administration of BHB at an early stage following status epilepticus the subsequent development of epileptogenesis could be prevented.

### REFERENCES

1 Vining EPG. Clinical efficacy of the ketogenic diet. Epilepsy Res. 1999; 37: 181-190.
2 Freeman JM, Vining EPG, Pyzik PL, Gilbert DL. Beta-hydroxybutyrate levels in blood correlate with seizure control in children on the ketogenic diet. Epilepsia 1998; 39 (suppl 6): 167-171.
3 Bough KJ, Eagles DA. A ketogenic diet increases the resistance to pentylenetetrazole-induced seizures in the rat. Epilepsia 1999; 40; 138-143.
4 Bough KJ, Valiyil R, Han FT, Eagles DA. Seizure resistance is dependent upon age and calorie restriction in rats fed a ketogenic diet. Epilepsy Res. 1999; 35: 21-28.
5 Ben-Ari Y. Limbic seizure and brain-damage produced by kainic acid: mechanisms and relevance to human temporal lobe epilepsy. Neuroscience 1985; 14: 375-403.
6 Lowenstein DH. Status epilepticus: an overview of the clinical problem. Epilepsia 1999; 40 (suppl 1): S3-S8.
7 Gähwiler BH. Organotypic monolayer cultures of nervous tissue. J. Neurosci. Methods 1981; 4: 329-342.
8 Gähwiler BH, Capogna M, Debanne D, McKinney RA, Thompson SM. Organotypic slice cultures: a technique has come of age. Trends Neurosci. 1997; 20 :471-477.
9 Veech RL, Chance B, Kashiwaya Y, Lardy HA, Cahill Jr GF. Ketone bodies, potential therapeutic uses. IUBMB Life 2001; 51; 241-247.
10 Sutula T, Cascino G, Cavazos J, Parada I, Ramirez L. Mossy fiber synaptic reorganization in the epileptic human temporal lobe. Annals of Neurol. 1989; 26; 321-330.
11 Babb TL, Kupfer WR, Pretorius JK, Crandall PH, Levesque MF. Synaptic reorganisation by mossy fibers in human epileptic fascia-dentata. Neuroscience 1991; 42; 351-363.
12 Esclapez M, Hirsch JC, Ben-Ari Y, Bernard C. Newly formed excitatory pathways provide a substrate for hyperexcitability in experimental temporal lobe epilepsy. J. Comp. Neurol. 1999; **408**; 449-460.
13 Fujikawa DG, Shinmei SS, Cai BY. Kainic acid-induced seizures produce necrotic, not apoptotic, neurons with internucleosomal DNA cleavage: implications for programmed cell death mechanisms. Neuroscience 2000; **98**; 41-53.
14 Ignatowicz E, Vezzani, AM, Rizzi M, D'Incalci M. Nerve cell death induced in vivo by kainic acid and quinolinic acid does not involve apoptosis. Neuroreport 1991; **2**; 651-654.
15 Pollard H, Charriaut-Marlangue C, Cantagrel S, Represa A, Robain O, Moreau J, Ben Ari Y. Kainate-induced apoptotic cell death in hippocampal neurons. Neuroscience 1994; **63**; 7-18.
16 Bengzon J, Kokaia Z, Elmer E, Nanobashvili A, Kokaia M, Lindvall O. Apoptosis and proliferation of dentate gyrus neurons after single and intermittent limbic seizures. Proc. Nat. Acad. Sci. 1997; **94**; 10432-10437.
17 Sakhi S, Bruce A, Sun N, Tocco G, Baudry M, Schreiber SS. Induction of tumor suppressor p53 and DNA fragmentation in organotypic hippocampal cultures following excitotoxin treatment. Exp. Neurol. 1997; **145**; 81-88.
18 Cheung N, Pascoe CJ, Giardina SF, John CA, Beart, PM. Micromolar L-glutamate induces extensive apoptosis in an apoptotic-necrotic continuum of insult-dependent, excitotoxic injury in cultured cortical neurones. Neuropharmacology 1998; 37; 1419-1429.
19 Venero JL, Revuelta M, Machado A, Cano J. Delayed apoptotic pyramidal cell death in CA4 and CA1 hippocampal subfields after a single intraseptal injection of kainate. Neuroscience 1999; 94; 1071-1081.
20 Kondratyev A, Gale K. Temporal and spatial patterns of DNA fragmentation following focally or systemically-evoked status epilepticus in rats. Neuroscience Letts. 2001; 310; 13-16.
21 Liu W, Liu RL, Chun JT, Bi RF, Hoe W, Schreiber SS, Baudry M. Kainate excitotoxicity in organotypic hippocampal slice cultures: evidence for multiple apoptotic pathways. Brain Res. 2001; 916; 239-248.
22 Arends MJ, Wyllie AH. Apoptosis: mechanisms and roles in the pathology. Int. Rev. Exp. Pathol. 1991; 32; 223-254.
23 Hu, Z, Yuri K, Ozawa H, Lu, H, Kawata M. The in vivo time course for elimination of adrenalectomy-induced apoptotic profiles from the granule cell layer of the rat hippocampus. J. Neurosci. 1997; 17; 3981-3989.
24 Leist MF, Gantner F, Bohlinger I, Germann PG, Tiegs G, Wendel A. TNF-induced murine hepatic apoptosis as a pathomechanism of septic liver failure. Am. J. Pathol. 1995; 166; 1-15.
25 Muller-Schwarze AB, Tandon P, Yang Y, Liu Z, Holmes, GL, Stafstrom CE. Ketogenic diet reduces spontaneous seizures and mossy fibre sprouting in the kainic acid model. Neuroreport 1999; 10; 1517-1522.
26 Nadler JV, Perry BW, Cotman CW. Selective reinnervation of hippocampal area CA1 and the fascia dentata after destruction of CA3-CA4 afferents with kainic acid. Brain Res. 1980; 182; 1-9.
27 de Lanerolle, NC, Kim JH, Robbins RJ, Spencer DD. Hippocampal interneuron loss and plasticity in human temporal lobe epilepsy. Brain Res. 1989; 495; 387-395.
28 Jagger, E. The effects of kainic acid in hippocampal organotypic cultures: a chronic in vitro model of temporal lobe epilepsy. PhD thesis, University of Nottingham 2002.

**TABLE 1**

| Material | Type | Reference |
|---|---|---|
| Sodium (R)-3-hydroxybutyrate | Salt | US 4579955 |
| | | US 4771074 |
| Dimer and | Metabolic | JP 5009185 |
| trimer BHB | precursor | JP 2885261 |
| Acetoacetyltri-3HB | Metabolic | US 6207856 |
| | precursor | |
| Triolide | Metabolic | WO 00/15216 |
| | precursor | WO 00/04895 |
| BHB-triglyceride | Metabolic | US 5420335 |
| | precursor | US 6306828 |
| BHB | Metabolic | WO 00/14985 |
| multimers | precursor | |

**TABLE 2: Experimental procedure for the assessment of neuronal cell death**

| Treatment | BHB (mM) | KA (µM) for 24 h | Propidium iodide (µM) | Addition of BHB after KA administration | Assessment of neuronal cell death | Number of cultures evaluated |
|---|---|---|---|---|---|---|
| Control | - | - | 2 | - | 24 h, 1 wk | 12 |
| KA only | - | 1 | 2 | - | 24 h, 1 wk | 12 |
| KTX0101 + KA | 0.3 | 1 | 2 | 30 mins, 24 h | 24 h, 1 wk | 12 |
| KTX0101 + KA | 1 | 1 | 2 | 30 mins, 24 h | 24 h, 1 wk | 13 |
| KTX0101 + KA | 3 | 1 | 2 | 30 mins, 24 h | 24 h, 1 wk | 13 |
| | | | | **Total no cultures:** | | **62** |

**TABLE 3: The effects of incubation of KTX0101 on kainic acid-induced status epilepticus at 24 h**

| **A) Comparison against control cultures** | | | | | |
|---|---|---|---|---|---|
| Treatment | n | Mean | SEM | 95% confidence interval | p |
| Control | 12 | 1.68 | 0.49 | | |
| KA 1 µM | 12 | 8.89 | 1.78 | -13.05, -1.37 | * |
| KTX0101 0.3 mM + KA 1 µM | 12 | 6.48 | 2.50 | -10.65, 1.04 | ns |
| KTX0101 1.0 mM + KA 1 µM | 13 | 4.89 | 1.45 | -8.94, 2.51 | ns |
| KTX0101 3.0 mM + KA 1 µM | 13 | 4.72 | 1.26 | -8.77,2.68 | ns |
| Each data point represents the mean ± S.E.M for the percentage propidium iodide uptake. Significant differences from the control cultures are denoted by *p<0.05 (one-way analysis of variance followed by Dunnett's multiple comparisons tests (two-tailed)). Ns = not significant. | | | | | |
| | | | | | |

| **B) Comparison against KA-treated cultures** | | | | | |
|---|---|---|---|---|---|
| Treatment | n | Mean | SEM | 95% confidence interval | p |
| KA 1 µM | 12 | 8.89 | 1.78 | | |
| KTX0101 0.3 mM + KA 1 µM | 12 | 6.48 | 2.50 | -3.44, 8.25 | ns |
| KTX0101 1.0 mM + KA 1 µM | 13 | 4.89 | 1.45 | -1.73, 9.73 | ns |
| KTX0101 3.0 mM + KA 1 µM | 13 | 4.72 | 1.26 | -1.56, 9.90 | ns |
| Each data point represents the mean ± S.E.M for the percentage propidium iodide uptake. Statistical comparisons between KA-treated and KTX0101-treated cultures were by one-way analysis of variance followed by Dunnett's multiple comparisons test (two-tailed). Ns = not significant. | | | | | |

**TABLE 4: The effects of incubation of KTX0101 on kainic acid-induced status epilepticus at 7 d**

| **A) Comparison against control cultures** | | | | | |
|---|---|---|---|---|---|
| Treatment | n | Mean | SEM | 95% confidence interval | p |
| Control | 12 | 1.81 | 0.99 | | |
| KA 1 µM | 12 | 6.97 | 2.03 | -8.78, -1.54 | *** |
| KTX0101 0.3 mM + KA 1 µM | 12 | 1.54 | 0.33 | -3.35, 3.90 | ns |
| KTX0101 1.0 mM + KA 1 µM | 13 | 1.05 | 0.29 | -2.27, 4.32 | ns |
| KTX0101 3.0 mM + KA 1 µM | 13 | 1.25 | 0.21 | -2.98, 4.13 | ns |
| Each data point represents the mean ± S.E.M for the percentage propidium iodide uptake. Significant differences from the control cultures are denoted by ***p<0.001 (one-way analysis of variance followed by Dunnett's multiple comparisons tests (two-tailed)). Ns = not significant. | | | | | |
| | | | | | |

| **B) Comparison against KA-treated cultures** | | | | | |
|---|---|---|---|---|---|
| Treatment | n | Mean | SEM | 95% confidence interval | p |
| KA 1 µM | 12 | 6.97 | 2.03 | | |
| KTX0101 0.3mM + KA 1 µM | 12 | 1.54 | 0.33 | 1.81,9.06 | *** |
| KTX0101 1.0 mM + KA 1 µM | 13 | 1.05 | 0.29 | 2.37, 9.48 | *** |
| KTX0101 3.0 mM + KA 1 µM | 13 | 1.25 | 0.21 | 2.18, 9.29 | *** |
| Each data point represents the mean ± S.E.M for the percentage propidium iodide uptake. Significant differences from the KA-treated cultures are denoted by ***p<0.001 (one-way analysis of variance followed by Dunnett's multiple comparisons tests (two-tailed)). | | | | | |

## Claims

1. A ketogenic material selected from (R)-3-hydroxybutyrate and its salts, esters and oligomers, for use in the treatment of acute intractible seizures wherein the treatment is intiated after onset of the seizures to arrest apoptosis.

2. A ketogenic material as claimed in Claim 1, for use in the treatment of status epilepticus wherein the treatment is initiated after onset of status epilepticus.

3. A ketogenic material for use in treatment as claimed in Claim 1 or Claim 2, wherein the treatment produces a ketosis in which total levels of acetoacetate and (R)-3-hydroxybutyrate concentrations in the blood of the subject are elevated above the normal fed levels to between 0.1 and 30mM.

4. A ketogenic material for use in treatment as claimed in Claim 3, characterised wherein the level is raised to between 0.3 and 15mM.

5. A ketogenic material for use in treatment as claimed in any one of Claims 1 to 4, **characterised in that** the treatment dose is in the range 5 to 5000mg/kg body weight.

6. A ketogenic material for use in treatment as claimed in any one of Claims 1 to 5, wherein the treatment administers the material to a patient unable to voluntarily admit materials by mouth.

7. A ketogenic material for use in treatment as claimed in any one of the preceeding claims, **characterised in that** the treatment administers the material by gastric feeding tube.

8. A ketogenic material for use in treatment as claimed in any one of the preceeding claims, **characterised in that** the material is administered as a parenteral solution intravenously, intraperitoneally or other systemic route.

## Patentansprüche

1. Ketogenes Material, das aus (R)-3-Hydroxybutyrat und seinen Salzen, Estern und Oligomeren ausgewählt ist, zur Verwendung bei der Behandlung schwerbehandelbarer epileptischer Anfälle, wobei die Behandlung nach Einsetzen der Anfälle begonnen wird, um Apoptose zu stoppen.

2. Ketogenes Material nach Anspruch 1 zur Verwendung bei der Behandlung eines Status epilepticus, wobei die Behandlung nach Einsetzen des Status epilepticus begonnen wird.

3. Ketogenes Material zur Verwendung bei der Behandlung nach Anspruch 1 oder Anspruch 2, wobei die Behandlung eine Ketose herstellt, bei der die Gesamtniveaus der Acetoacetat- und (R)-3-Hydroxybutyrat-Konzentrationen im Blut des Subjekts über die normalen zugeführten Niveaus auf zwischen 0,1 und 30 mmol erhöht sind.

4. Ketogenes Material zur Verwendung bei der Behandlung nach Anspruch 3, wobei es **dadurch gekennzeichnet ist, dass** das Niveau auf zwischen 0,3 und 15 mmol angehoben ist.

5. Ketogenes Material zur Verwendung bei der Behandlung nach einem der Ansprüche 1 bis 4, **dadurch gekennzeichnet, dass** die Behandlungsdosis im Bereich 5 bis 5000 mg/kg Körpergewicht liegt.

6. Ketogenes Material zur Verwendung bei der Behandlung nach einem der Ansprüche 1 bis 5, wobei die Behandlung das Material einem Patienten verabreicht, der nicht in der Lage ist, freiwillig Materialien über den Mund zuzuführen.

7. Ketogenes Material zur Verwendung bei der Behandlung nach einem der vorhergehenden Ansprüche, **dadurch gekennzeichnet, dass** die Behandlung das Material durch eine Magensonde verabreicht.

8. Ketogenes Material zur Verwendung bei der Behandlung nach einem der vorhergehenden Ansprüche, **dadurch gekennzeichnet, dass** das Material als eine parenterale Lösung intravenös, intraperitoneal oder auf anderem systemischen Weg verabreicht wird.

## Revendications

1. Matériau cétogène sélectionné parmi (R)-3-hydroxybutyrate et ses sels, esters et oligomères, pour une utilisation dans le traitement de crises épileptiques réfractaires aiguës dans lequel le traitement est débuté après l'apparition des crises épileptiques pour enrayer l'apoptose.

2. Matériau cétogène selon la revendication 1, pour une utilisation dans le traitement de l'état de mal épileptique dans lequel le traitement est débuté après l'apparition de l'état de mal épileptique.

3. Matériau cétogène pour une utilisation dans le traitement selon la revendication 1 ou la revendication 2, dans lequel le traitement produit une cétose dans laquelle les niveaux totaux de concentrations en acétoacétate et en (R)-3-hydroxybutyrate dans le sang du sujet sont amenés au-dessus des niveaux normaux à une valeur comprise entre 0,1 et 30 mM.

4. Matériau cétogène pour une utilisation dans le traitement selon la revendication 3, **caractérisé en ce que** le niveau est amené à une valeur comprise entre 0,3 et 15 mM.

5. Matériau cétogène pour une utilisation dans le traitement selon l'une quelconque des revendications 1 à 4, **caractérisé en ce que** la dose de traitement se situe dans la plage allant de 5 à 5 000 mg/kg de masse corporelle.

6. Matériau cétogène pour une utilisation dans le traitement selon l'une quelconque des revendications 1 à 5, dans lequel le traitement administre le matériau à un patient incapable de laisser entrer volontairement des matériaux par la bouche.

7. Matériau cétogène pour une utilisation dans le traitement selon l'une quelconque des revendications précédentes, **caractérisé en ce que** le traitement administre le matériau par sonde gastrique.

8. Matériau cétogène pour une utilisation dans le traitement selon l'une quelconque des revendications précédentes, **caractérisé en ce que** le matériau est administré en tant que solution parentérale par voie intraveineuse, par voie intrapéritonéale ou par une autre voie systémique.
